**Europäisches Patentamt**

**European Patent Office**

**Office européen des brevets**

(11) Veröffentlichungsnummer: **0 281 647 B1**

(12) **EUROPÄISCHE PATENTSCHRIFT**

(45) Veröffentlichungstag der Patentschrift: **07.10.92**

(51) Int. Cl.⁵: **A61K 7/48**

(21) Anmeldenummer: **87103398.1**

(22) Anmeldetag: **10.03.87**

(54) **Organische Verbindungen zur Vergrösserung der weiblichen Brust.**

(43) Veröffentlichungstag der Anmeldung:
**14.09.88 Patentblatt 88/37**

(45) Bekanntmachung des Hinweises auf die Patenterteilung:
**07.10.92 Patentblatt 92/41**

(84) Benannte Vertragsstaaten:
**AT BE CH DE ES FR GB GR IT LI LU NL SE**

(56) Entgegenhaltungen:
**FR-A- 1 438 314**
**FR-A- 2 296 425**

(73) Patentinhaber: **von Sonnleithner, Michael, Dr. med.**
**Grosse Strasse 9A**
**W-2070 Ahrensburg(DE)**

(72) Erfinder: **von Sonnleithner, Michael, Dr. med.**
**Grosse Strasse 9A**
**W-2070 Ahrensburg(DE)**

(74) Vertreter: **Schöning, Hans-Werner, Dipl.-Ing.**
**Patentanwälte Niedmers & Schöning Jessenstrasse 4**
**W-2000 Hamburg 50(DE)**

## Beschreibung

Die Erfindung betrifft ein Mittel zur Vergrößerung der Brust des Menschen sowie die Verwendung eines derartigen Mittels zu diesem Zwecke,
Es ist bekannt, in der plastischen Chirurgie operative Eingriffe zur Vergrößerung der weiblichen Brust vorzunehmen, wobei Implantate aus Silikon oder ähnlichen Verbindungen eingesetzt werden. Auch ist es bekannt, die interne und externe Applikation von hormonhaltigen Prä-paraten zu einer Vergrößerung der weiblichen Brust vorzunehmen.

Nachteilig ist aber bei diesen bekannten unnatürlichen Methoden, daß sie einerseits gewebezerstörend wirken und andererseits Nebenwirkungen zeigen, die vermieden werden sollten.

Es ist Aufgabe der vorliegenden Erfindung, ein Mittel zur Vergrößerung der weiblichen Brust zu schaffen, das einerseits für den Organismus des Menschen gut verträglich ist, das andererseits sehr wirksam ist und schließlich risikobehaftete, operative Eingriffe entbehrlich macht und bei gezieltem Einsatz lediglich, wie gewünscht, die Brust maß- und wunschgerecht vergrößert.

Gelöst wird die Aufgabe gemäß der Erfindung durch die Kombination folgender Verbindungen: Triglyceride der Ölsäure, Stearinsäure, Palmitinsäure, Linolsäure sowie 10-20 % Paraffinum Perliquidum, bezogen auf das Gewicht der gesamten Mischung.

Der Vorteil des Mittels besteht im wesentlichen in der grundsätzlich leicht zu applizierenden Form des Mittels, wobei es sich bei den Triglyceriden um sehr körperfreundliche Stoffe handelt, die in Venen und u.a. über die Schweißdrüsen der Achselhöhle aufgenommen werden. Die Substanzen werden in das Fettgewebe der Brust aufgenommen und abgelagert. Außerdem wird dadurch der Lymph- und der Venenabfluß geringfügig verlangsamt, was dem Wachstum der Brust zugute kommt.

Es hat sich herausgestellt, daß gemäß einer vorteilhaften Ausgestaltung des Mittels die Verbindungen in folgenden Gewichtsverhältnissen vorliegen: die Triglyceride enthalten 25-35 % Ölsäure, 25-35 % Stearinsäure, 15-25 % Palmitinsäure und 1-10 % Linolsäure. Insbesondere bei den vorgenannten Gewichtsverhältnissen des Mittels hat sich gezeigt, daß nach Massage mit diesem Mittel der von der Brust wegführenden Venen und Lymphgefäße, die der Drainage der Brust und der Achselhöhlenschweißausführungsgänge dienen, eine meßbare Vergrößerung des Brustumfanges von 3 bis 5 cm festgestellt wurde.

Um das Mittel auch für die Anwendung attraktiv zu machen, umfaßt dieses vorteilhafterweise auch einen oder mehrere Duftstoffe.

Aus der FR-A- 1 438 314 ist ein Mittel zur Schönheitspflege bekannt, das als Flüssigkeit, Creme oder in pulverförmiger Form auftragbar ist und Glycole enthält.

Aus der FR-A- 2 296 425 ist ein Mittel zur äußeren Anwendung zur Behandlung der weiblichen Brust nach der Methode nach Filatov bekannt, bei dem unter anderem ebenfalls Glycole verwendet werden.

Die Erfindung wird nun unter Bezugnahme auf die einzige schematische Zeichnung anhand eines Ausführungsbeispieles beschrieben. Diese zeigt:
In der Teilansicht eine Draufsicht auf den Oberkörper bzw. die Brust eines weiblichen Menschen, bei der die Zonen zum Auftragen und Einmassieren des Mittels hervorgehoben sind.

Um die vorteilhafteste Wirkung der Mischung von Triglyceriden zu finden, die eine Vergrößerung der weiblichen Brust bewirken sollen, wurde eine Versuchsgruppe von 20 Frauen im Alter von 16 bis 39 Jahren hinzugezogen. Zwischen den Brüsten und an den Flächen entlang der Brust unterhalb der Achselhöhlen, wo sich die Arterien und Venen mammariae internae und externae befinden, wurde mit einer Mischung von körperfreundlichen Triglyzeriden und Paraffinum Perliquidum über einen Zeitraum von 8 Wochen einmassiert. Es wurde mit verschiedenen Prozentsätzen der Triglyceride experimentiert.

Eine Verbindung mit folgenden Gewichtsprozenten zeigte die besten Ergebnisse: die Triglyceride enthalten 25 - 35 % Ölsäure, 25 - 35 % Stearinsäure, 15 - 25 % Palmitinsäure und 1 - 10 % Linolsäure.

Nach 8 Wochen Massage mit einer dieses Mittel enthaltenden Hartcreme auf die vorgegebenen Stellen zeigte sich bei der Versuchsgruppe der Frauen in allen Fällen eine meßbare Vergrößerung des Brustumfangs von mindestens 3 - 5 cm, die sich bei weiterer Behandlung mit dem Mittel fortsetzte.

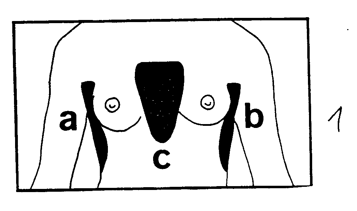

## Patentansprüche

1. Mittel zur Vergrößerung der weiblichen Brust, gekennzeichnet durch die Kombination folgender Verbindungen: Triglyceride der Ölsäure, Stearinsäure, Palmitinsäure, Linolsäure sowie 10-20 % Paraffinum Perliquidum, bezogen auf das Gewicht der gesamten Mischung.

2. Mittel nach Anspruch 1, dadurch gekennzeichnet, daß die Verbindungen in folgenden Gewichtsverhältnissen vorliegen: die organischen Verbindungen aus Glycerin und Fettsäuren enthalten 25-23 % Ölsäure, 25-35 % Stearinsäure, 15-25 % Palmitinsäure, 1-10 % Linolsäure.

3. Mittel nach Anspruch 1 oder 2, dadurch gekennzeichnet, daß das Gemisch wenigstens einen Duftstoff umfaßt.

4. Verwendung des Mittels nach Anspruch 1 zur Vergrößerung der weiblichen Brust

**Claims**

1. An agent for enlarging the female breast, characterised by the combination of the following compounds: triglycerides of oleic acid, stearic acid, palmitic acid, linoleic acid together with 10-20% paraffinum perliquidum, in relation to the total weight of the mixture.

2. An agent according to Claim 1, characterised in that the compounds are present in the following proportions by weight: the organic compounds of glycerine and fatty acids contain 25-23% oleic acid, 25-35% stearic acid, 15-25% palmitic acid and 1-10% linoleic acid.

3. An agent according to Claim 1 or 2, characterised in that the mixture contains at least one aromatic substance.

4. Use of the agent according to Claim 1 for enlargement of the female breast.

**Revendications**

1. Substance pour développer les seins, caractérisée par la combinaison de composés suivants: triglycérides de l'acide oléique, de l'acide stéarique, de l'acide palmitique, de l'acide linolinéique ainsi que 10-20 % de Paraffinum-Perliquidum, rapportés au poids de l'ensemble du mélange.

2. Substance selon la revendication 1, caractérisé en ce que les composés sont présents avec les rapport pondéraux suivants: les composés organiques à base de glycérine et d'acides gras contiennent 25-23 % d'acide oléique, 25-35 % d'acide stéarique, 15-25 % d'acide palmitique, 1-10 % d'acide linoléique.

3. Substance selon la revendication 1 ou 2, caractérisée en ce que le mélange contient au moins une substance odoriférante.

4. Utilisation de la substance selon la revendication 1 pour développer les seins.

EP 0 281 647 B1

4